(19) **Europäisches Patentamt / European Patent Office / Office européen des brevets**

(11) **EP 3 954 289 A1**

(12) **EUROPEAN PATENT APPLICATION**

(43) Date of publication:
**16.02.2022 Bulletin 2022/07**

(21) Application number: **21191412.2**

(22) Date of filing: **13.08.2021**

(51) International Patent Classification (IPC):
**A61B 5/1455** (2006.01) **A61B 5/0215** (2006.01)
**A61B 5/0245** (2006.01) **A61B 5/00** (2006.01)
**A61B 5/352** (2021.01)

(52) Cooperative Patent Classification (CPC):
**A61B 5/14551; A61B 5/0215; A61B 5/0245;**
**A61B 5/352; A61B 5/7221; A61B 5/7246;**
**A61B 5/7275;** A61B 2562/06

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **13.08.2020 CN 202010817171**

(71) Applicant: **Shenzhen Mindray Bio-Medical**
**Electronics Co., Ltd.**
**Shenzhen, Guangdong 518057 (CN)**

(72) Inventors:
- **SU, Jianwei**
  **Shenzhen, 518057 (CN)**
- **SUN, Zehui**
  **Shenzhen, 518057 (CN)**
- **YE, Wenyu**
  **Shenzhen, 518057 (CN)**
- **GUAN, Zehong**
  **Shenzhen, 518057 (CN)**
- **YANG, Kang**
  **Shenzhen, 518057 (CN)**

(74) Representative: **KIPA AB**
**Drottninggatan 11**
**252 21 Helsingborg (SE)**

(54) **MONITORING DEVICE AND PHYSIOLOGICAL PARAMETER PROCESSING METHOD THEREFOR**

(57)     The disclosure provides a physiological parameter processing method, including: obtaining at least two physiological signals; respectively processing the at least two physiological signals, to obtain physiological parameters of a same type that are derived from the physiological signals; respectively analysing the at least two physiological signals, to obtain correlation coefficients of the physiological signals and corresponding historical physiological signals; and fusing, according to the obtained correlation coefficients of the physiological signals and the corresponding historical physiological signals, the physiological parameters of the same type that are derived from the physiological signals, to obtain a fused physiological parameter. The disclosure further provides a monitoring device. The disclosure implements the fusion of the physiological parameters of the same type of the plurality of physiological signals, thereby avoiding erroneous determination.

EP 3 954 289 A1

**Description**

**TECHNICAL FIELD**

The disclosure relates to a monitoring device, and in particular to a monitoring device and a physiological parameter processing method therefor.

**BACKGROUND**

In conventional physiological parameter processing strategies, research and improvement are made on the basis of a single physiological parameter, to improve estimation of a physiological parameter of a single physiological signal in the presence of noise. The strategies are, for example, classical filtering, machine learning, Kalman filtering, and transform domain analysis. However, none of the technologies can well address impact of noise. In addition, some algorithms are complex in computing and require a large amount of resources, but can hardly perform real-time computing and engineering implementation.

**SUMMARY**

Embodiments of the disclosure disclose a monitoring device and a physiological parameter processing method therefor, which can avoid issues caused by computing based on a single physiological parameter, so as solve the above-mentioned problems.

According to a first aspect, an embodiment of the disclosure discloses a physiological parameter processing method, including:

obtaining at least two physiological signals;
respectively processing each physiological signal, to obtain a physiological parameter that is respectively derived from each physiological signal, wherein the obtained physiological parameters of the at least two physiological signals are of a same type;
respectively analysing each physiological signal, to obtain a correlation coefficient between each physiological signal and its corresponding historical physiological signal; and
fusing the obtained physiological parameters of the same type to obtain a fused physiological parameter, according to the obtained correlation coefficients.

In an embodiment of the invention, fusing the obtained physiological parameters of the same type to obtain a fused physiological parameter, according to the obtained correlation coefficients comprises:

determining a weight of each physiological parameter of the same type, according to each correlation coefficient corresponding to each physiological signal; and
fusing the obtained physiological parameters of the same type by adopting a weighted averaging method for the at least two physiological parameters of the same type and their at least two weights, so as to obtain the fused physiological parameter.

In another embodiment of the invention, determining a weight of each physiological parameter of the same type, according to each correlation coefficient corresponding to each physiological signal comprises:

obtaining a pre-set correspondence table of correlation coefficients and weights; and
obtaining a weight of each physiological parameter of the same type from the correspondence table, according to each correlation coefficients.

In another embodiment of the invention, a weight of a physiological parameter of the same type from a physiological signal with a higher correlation coefficient is greater than a weight of a physiological parameter of the same type from a physiological signal with a lower correlation coefficient.

In yet another embodiment of the invention, respectively analysing each physiological signal, to obtain a correlation coefficient between each physiological signal and its corresponding historical physiological signal comprises:

obtaining a waveform segment of each physiological signal that is used to obtain the physiological parameter of the same type; and
respectively performing a correlation coefficient calculation, according to each waveform segment and a historical physiological signal corresponding to each physiological signal, so as to obtain a correlation coefficient between each physiological signal and its corresponding historical physiological signal.

In yet another embodiment of the invention, respectively performing a correlation coefficient calculation, according to each waveform segment and a historical physiological signal corresponding to each physiological signal, so as to obtain a correlation coefficient between each physiological signal and its corresponding historical physiological signal comprises:

obtaining a historical waveform segment of each physiological signal that is used to obtain a historical physiological parameter of a same type from the historical physiological signal of each physiological signal;
obtaining a fluctuation index of each physiological signal, according to a similarity between the waveform segment of each physiological signal and the historical waveform segment of each physiological signal, and
obtaining a correlation coefficient between each physiological signal and its corresponding historical physiological signal, according to the fluctuation index of each physiological signal.

In another embodiment of the invention, respectively analysing each physiological signal, to obtain a correlation coefficient between each physiological signal and its corresponding historical physiological signal comprises:

obtaining, from each physiological signal, a physiological parameter of the same type of each physiological signal within a first pre-set time period;
obtaining, from a historical physiological signal of each physiological signal, a historical trend of each physiological parameter of the same type of each physiological signal within a second pre-set time period; and
obtaining a fluctuation index of each physiological parameter of the same type, according to a similarity between each physiological parameter of the same type of each physiological signal and a historical trend of each physiological parameter of the same type of a historical physiological signal of each physiological signal, and
obtaining a correlation coefficient between each physiological signal and its corresponding historical physiological signal, according to the fluctuation index of each physiological parameter of the same type.

In another embodiment of the invention, the historical trend of each physiological parameter of the same type comprise a historical average of each physiological parameter of the same type, or a historical average of a fused physiological parameter obtained from the historical physiological signals of the at least two physiological signals.

In yet another embodiment of the invention, respectively analysing each physiological signal, to obtain a correlation coefficient between each physiological signal and its corresponding historical physiological signal comprises:

obtaining, from each physiological signal, a physiological parameter of the same type of each physiological signal within a pre-set time period;
obtaining, from a historical physiological signal of each physiological signal, a physiological parameter of the same type of the historical physiological signal of each physiological signal; and
obtaining a fluctuation index of each physiological parameter, according to a statistical relationship between an average and a deviation of each physiological parameter of the same type of each physiological signal and each physiological parameter of the same type of the historical physiological signal of each physiological signal, and
obtaining a correlation coefficient between each physiological signal and its corresponding historical physiological signal, according to the fluctuation index of each physiological parameter.

In yet another embodiment of the invention, respectively analysing each physiological signal, to obtain a correlation coefficient between each physiological signal and its corresponding historical physiological signal comprises:

determining a number of a noise alarm of each physiological signal within a pre-set time period, according to each physiological signal and a historical physiological signal of each physiological signal, and
determining a correlation coefficient between each physiological signal and its corresponding historical physiological signal, according to the number of the noise alarm of each physiological signal.

In another embodiment of the invention, respectively analysing each physiological signal, to obtain a correlation coefficient between each physiological signal and its corresponding historical physiological signal comprises:

determining a number of time during which each parameter of the same type is invalid within a pre-set time period, according to each physiological signal and a historical physiological signal of each physiological signal, and
determining a correlation coefficient between each physiological signal and its corresponding historical physiological signal, according to the number of time during which each parameter of the same type is invalid.

In another embodiment of the invention, the at least two physiological signals comprise at least two of an ECG signal, an IBP signal, and an Sp02 signal; and the at least two physiological parameters of the same type are at least two of an ECG heart rate of the ECG signal, an IBP pulse rate of the IBP signal, and an Sp02 pulse rate of the Sp02 signal.

In yet another embodiment of the invention, respectively processing each physiological signal, to obtain a physiological parameter of a same type comprises:

respectively processing the at least two of the ECG signal, the IBP signal, and the SpO2 signal to obtain the at least two of the ECG heart rate of the ECG signal, the IBP pulse rate of the IBP signal, and the SpO2 pulse rate of the SpO2 signal;
respectively analysing the at least two physiological signals, to obtain at least two correlation coefficients between the at least two physiological signals and their corresponding at least two historical physiological signals comprises:

respectively analysing the at least two of the ECG signal, the IBP signal, and the SpO2 signal to obtain at least two of a first correlation coefficient between an ECG waveform of the ECG signal and a historical ECG waveform of the ECG signal, a second correlation coefficient between an IBP waveform of the IBP signal and a historical IBP waveform of the IBP signal, and a third correlation coefficient between an SpO2 waveform of the SpO2 signal and a historical SpO2 waveform of the SpO2 signal; and
fusing the at least two physiological parameters of the same type to obtain a fused physiological parameter, according to the obtained at least two correlation coefficients comprises:
obtaining a fused heart rate, according to the at least two of the first correlation coefficient, the second correlation coefficient, and the third correlation coefficient.

In another embodiment of the invention, respectively processing the at least two physiological signals, to obtain at least two physiological parameters of a same type that are respectively derived from the at least two physiological signals comprises:

respectively processing the at least two of the ECG signal, the IBP signal, and the SpO2 signal to obtain at least two of the ECG heart rate of the ECG signal, the IBP pulse rate of the IBP signal, and the SpO2 pulse rate of the SpO2 signal;
respectively analysing the at least two physiological signals, to obtain at least two correlation coefficients between the at least two physiological signals and their corresponding at least two historical physiological signals comprises:

respectively analysing the at least two of the ECG signal, the IBP signal, and the SpO2 signal to obtain at least two of a first correlation coefficient between the ECG heart rate of the ECG signal and a historical average ECG heart rate of the ECG signal, a second correlation coefficient between the IBP pulse rate of the IBP signal and a historical average IBP pulse rate of the IBP signal, and a third correlation coefficient between the SpO2 pulse rate of the SpO2 signal and a historical SpO2 pulse rate of the SpO2 signal; and
fusing the at least two physiological parameters of the same type that are respectively derived from the at least two physiological signals, according to the obtained at least two correlation coefficients between the at least two physiological signals and their corresponding at least two historical physiological signals, to obtain a fused physiological parameter comprises:
fusing at least two of the ECG heart rate of the ECG signal, the IBP pulse rate of the IBP signal, and the SpO2 pulse rate of the SpO2 signal, according to at least two of the first correlation coefficient between the ECG heart rate of the ECG signal and the historical average ECG heart rate of the ECG signal, the second correlation coefficient between the IBP pulse rate of the IBP signal and the historical average IBP pulse rate of the IBP signal, and the third correlation coefficient between the SpO2 pulse rate of the SpO2 signal and the historical average SpO2 pulse rate of the SpO2 signal, so as to obtain a fused heart rate.

In another embodiment of the invention, respectively processing the at least two physiological signals, to obtain at least two physiological parameters of the same type that are respectively derived from the at least two physiological signals comprises:

respectively processing at least two of the ECG signal, the IBP signal, and the SpO2 signal to obtain at least two of the ECG heart rate of the ECG signal, the IBP pulse rate of the IBP signal, and the SpO2 pulse rate of the SpO2 signal;
respectively analysing the at least two physiological signals, to obtain at least two correlation coefficients between the at least two physiological signals and their corresponding at least two historical physiological signals comprises:

respectively analysing the at least two of the ECG signal, the IBP signal, and the SpO2 signal to obtain at least two of a first correlation coefficient between the ECG heart rate of the ECG signal and a historical average fused heart rate, a second correlation coefficient between the IBP pulse rate of the IBP signal and the historical average fused heart rate, and a third correlation coefficient between the SpO2 pulse rate of the SpO2 signal and the historical average fused heart rate; and

fusing the at least two physiological parameters of the same type that are respectively derived from the at least two physiological signals, according to the obtained at least two correlation coefficients between the at least two physiological signals and their corresponding at least two historical physiological signals, to obtain a fused physiological parameter comprises:

fusing the at least two of the ECG heart rate of the ECG signal, the IBP pulse rate of the IBP signal, and the SpO2 pulse rate of the SpO2 signal, according to at least two of the first correlation coefficient between the ECG heart rate of the ECG signal and the historical average fused heart rate, the second correlation coefficient between the IBP pulse rate of the IBP signal and the historical average fused heart rate, and the third correlation coefficient between the SpO2 pulse rate of the SpO2 signal and the historical average fused heart rate, so as to obtain a fused heart rate.

In this way, physiological parameters of the same type that are derived from different signals can be fused for analysis to improve the accuracy of results of these physiological parameters, and when a single physiological signal is subjected to interference, physiological parameters from other physiological signals can be used for improvement, thereby avoiding the disadvantages of obtaining physiological parameters only on the basis of a single physiological signal.

## BRIEF DESCRIPTION OF THE DRAWINGS

To more clearly describe the technical solutions in the embodiments of the disclosure, the drawings required for describing the embodiments will be briefly described below. Apparently, the drawings in the following description show only some of the embodiments of the disclosure, and persons of ordinary skill in the art may still derive other drawings from these drawings without creative efforts.

FIG. 1 is a schematic flowchart of a physiological parameter processing method applied to a monitoring device according to an embodiment of the disclosure;
FIG. 2 is a schematic flowchart of a physiological parameter processing method applied to a monitoring device according to a first embodiment of the disclosure;
FIG. 3 is a schematic flowchart of a physiological parameter processing method applied to a monitoring device according to a second embodiment of the disclosure;
FIG. 4 is a schematic flowchart of a physiological parameter processing method applied to a monitoring device according to a third embodiment of the disclosure;
FIG. 5 is a schematic diagram of a respiratory baseline according to an embodiment of the disclosure; and
FIG. 6 is a schematic modular diagram of a monitoring device according to an embodiment of the disclosure.

## DESCRIPTION OF THE PREFERRED EMBODIMENTS

The technical solutions of the embodiments of the disclosure will be described below clearly and comprehensively in conjunction with accompanying drawings of the embodiments of the disclosure. Apparently, the embodiments described are merely some of, rather than all of, the embodiments of the disclosure. All other embodiments obtained by persons of ordinary skill in the art based on the embodiments of the disclosure without creative efforts shall fall within the scope of protection of the disclosure.

The terms "first", "second", etc. in the specification and the claims of the disclosure as well as the above accompanying drawings are used to distinguish different objects, rather than to describe a specific order. In addition, the term "include" and any variations thereof are intended to cover non-exclusive inclusion. For example, a process, a method, a system, a product, or a device that includes a series of steps or units is not limited to the listed steps or units, but optionally further includes unlisted steps or units, or optionally further includes other steps or units inherent to the process, the method, the product, or the device.

Preferred embodiments of the disclosure are subsequently described in the specification, but the description is for the purpose of explaining the general principles of the disclosure and is not intended to limit the scope of the disclosure. The scope of protection of the disclosure should be defined by the appended claims.

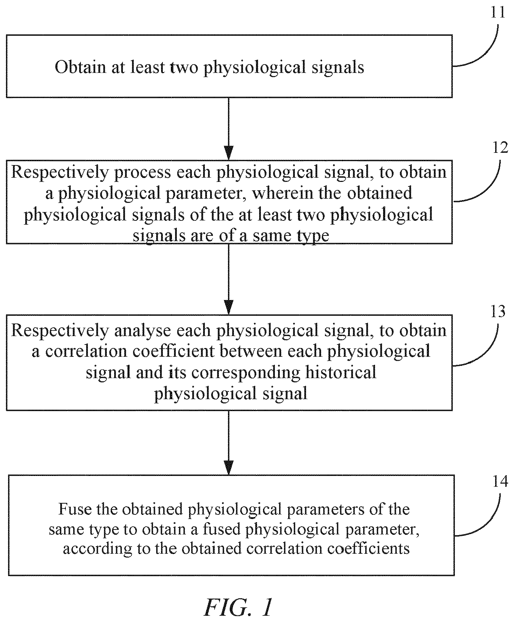

Referring to FIG. 1, FIG. 1 is a schematic modular diagram of a physiological parameter processing method applied to a monitoring device according to an embodiment of the disclosure. It can be understood that the order of steps of the physiological parameter processing method can be adjusted. Specifically, the physiological parameter processing method includes the following steps:

step 11: obtaining at least two physiological signals (such as an electrocardiogram signal (hereinafter referred to as an "ECG signal"), an invasive blood pressure signal (hereinafter referred to as an "IBP signal"), or an oxygen saturation signal (hereinafter referred to as an "SpO2 signal"));

step 12: respectively processing the at least two physiological signals, to obtain physiological parameters of a same type that are derived from the physiological signals (such as an ECG heart rate derived from the ECG signal, an IBP pulse rate derived from the IBP signal, or an SpO2 pulse rate derived from the SpO2 signal);

step 13: respectively analysing the at least two physiological signals, to obtain correlation coefficients of the physiological signals and corresponding historical physiological signals; and

step 14: fusing, according to the obtained correlation coefficients of the physiological signals and the corresponding historical physiological signals, the physiological parameters of the same type that are derived from the physiological signals, to obtain a fused physiological parameter.

In the disclosure, at least two types of physiological parameters of the same type that are derived from different signals can be fused for analysis to improve the accuracy of a fused physiological parameter that is finally output, and when a single physiological signal is subjected to interference, physiological parameters of the same type from other physiological signals can be used for improvement, thereby avoiding the disadvantages of obtaining physiological parameters only on the basis of a single physiological signal.

Further, in one embodiment, the correlation coefficients are the correlation coefficients of the physiological signals and the corresponding historical physiological signals. The correlation coefficient indicates the magnitude of correlation between each of the physiological signals and a corresponding historical physiological signal. The closer each of the physiological signals is to the corresponding historical physiological signal, the higher the correlation coefficient thereof; and conversely, the farther each of the physiological signals is from the corresponding historical physiological signal, the lower the correlation coefficient thereof.

Specifically, in one embodiment, the fusing, according to the obtained correlation coefficients of the physiological signals and the corresponding historical physiological signals, the physiological parameters of the same type that are derived from the physiological signals, to obtain a fused physiological parameter includes:

determining, according to the correlation coefficients of the physiological signals and the corresponding historical physiological signals, weights of the physiological parameters of the same type that are derived from the physiological signals; and

fusing the at least two physiological parameters of the same type by adopting a weighted averaging method for the at least two physiological parameters of the same type and their at least two weights, so as to obtain the fused physiological parameter.

In this way, the weights of the physiological parameters of the same type from the physiological signals can be determined from a change trend of the physiological signals with respect to their respective historical physiological signals, and physiological parameter errors caused by transient interference that often occurs in clinical practice can be effectively addressed. For example, an ECG heart rate suddenly changes due to the pulling of ECG electrode pads on a patient, but an SpO2 signal and an IBP signal are not affected and are very clean at the moment, and an SpO2 pulse rate and an IBP pulse rate respectively calculated on the basis of the SpO2 signal and the IBP signal are very stable. Therefore, during the calculation of a fused heart rate, a weight of the ECG heart rate may be reduced, and weights of the IBP pulse rate and the SpO2 pulse rate may be increased, such that the accuracy of the fused heart rate is ensured.

Further, in one embodiment, the determining, according to the correlation coefficients of the physiological signals and the corresponding historical physiological signals, weights of the physiological parameters of the same type that are derived from the physiological signals specifically includes:

obtaining a pre-set correspondence table of correlation coefficients and weights; and

obtaining, from the correspondence table according to the correlation coefficients of the physiological signals and the corresponding historical physiological signals, the weights of the physiological parameters of the same type that are derived from the physiological signals.

Therefore, the correspondence table of the correlation coefficients and the weights may be pre-established, and when the correlation coefficients of the physiological signals and the corresponding historical physiological signals are determined, the respective weights of the physiological signals are determined according to the correlation coefficients of the physiological signals and the corresponding historical physiological signals, thereby simplifying the process of determining the respective weights of the physiological signals.

Further, in one embodiment, a weight of a physiological parameter of the same type from a physiological signal with a higher correlation coefficient is greater than a weight of a physiological parameter of the same type from a physiological signal with a lower correlation coefficient.

Therefore, physiological parameters of the same type from other physiological signals may be used for improvement of a physiological signal which is subjected to interference, thereby avoiding the disadvantages of obtaining physiological parameters only on the basis of a single physiological signal.

Further, in one embodiment, the weights of the physiological parameters of the same type can be obtained according to the correlation coefficients of the physiological signals and the corresponding historical signals. It can be understood that the correlation coefficient may indicate a similarity between an original waveform segment of each of the physiological signals and an original waveform segment of the corresponding historical physiological signal, or a similarity between physiological parameters of the same type that are respectively derived from original waveform segments of each of the physiological signals and the corresponding historical physiological signal.

Specifically, in one embodiment, the respectively analysing the at least two physiological signals, to obtain correlation coefficients of the physiological signals and corresponding historical physiological signals includes:

> obtaining waveform segments of the physiological signals that are used to obtain the physiological parameters of the same type; and
> performing correlation coefficient calculation according to the waveform segments of the physiological signals that are used to obtain the physiological parameters of the same type and the historical signals corresponding to the physiological signals, so as to obtain the correlation coefficients of the physiological signals and the corresponding historical physiological signals.

Therefore, the correlation coefficient calculation is performed according to the waveform segments of the physiological signals that are used to obtain the physiological parameters of the same type and the historical signals corresponding to the physiological signals, and the correlation coefficients of the physiological signals and the corresponding historical physiological signals can be obtained.

Specifically, in a first embodiment, the historical signal is a historical waveform segment. The performing correlation coefficient calculation according to the waveform segments of the physiological signals that are used to obtain the physiological parameters of the same type and the historical signals corresponding to the physiological signals, so as to obtain the correlation coefficients of the physiological signals includes:

> obtaining, from the historical signals of the physiological signals, historical waveform segments of the historical signals that are used to obtain historical physiological parameters of the same type; and
> obtaining a fluctuation index of each physiological signal according to a similarity between the waveform segment of each physiological signal and the historical waveform segment of each physiological signal, and obtaining the correlation coefficient of each physiological signal and the corresponding historical physiological signal according to the fluctuation index of each physiological signal. Comparing the similarity between the waveform segment of each physiological signal and the historical waveform segment of each physiological signal includes comparing related parameter characteristic values or waveform patterns of the waveform segment of each physiological signal and the historical waveform segment of each physiological signal.

Therefore, by means of the correlation coefficients of the physiological signals and the historical physiological signals, weight values of the physiological parameters of the same type from the physiological signals are obtained, and interference from one or two physiological signals to a final result of the physiological parameters due to noise interference is reduced by changing the magnitude of the weight values.

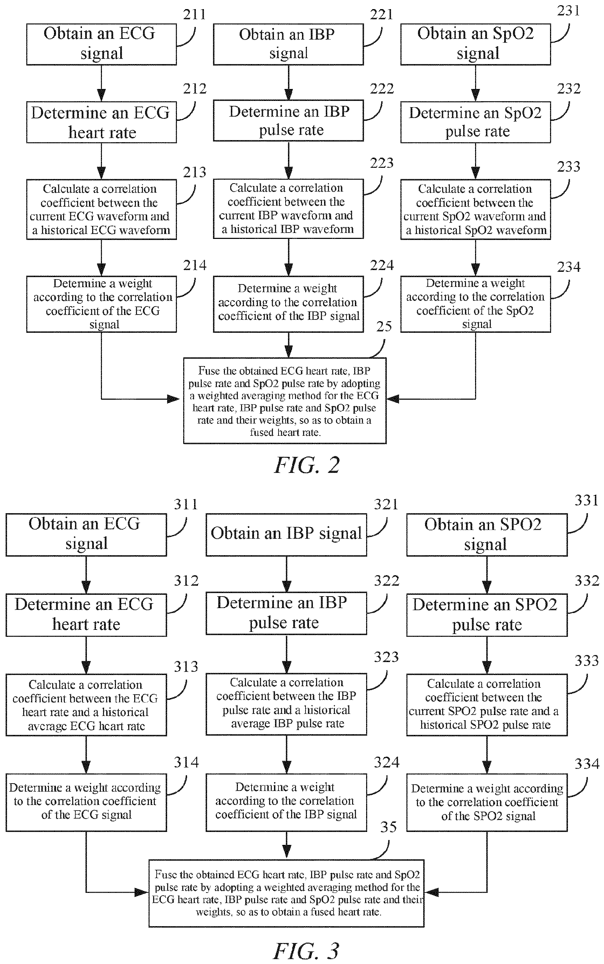

Referring to FIG. 2, in a first specific embodiment, the at least two physiological signals include three physiological signals, which are specifically an ECG signal, an IBP signal, and an SpO2 signal. The three physiological signals are all derived from the periodic beating of the heart. Therefore, an ECG heart rate, an IBP pulse rate, and an SpO2 pulse rate respectively derived from the three physiological signals are strongly correlated with each other, and the ECG heart rate, the IBP pulse rate, and the SpO2 pulse rate may be referred to as physiological parameters of the same type.

Therefore, the step of "obtaining at least two physiological signals" specifically includes:

> obtaining an ECG signal (step 211), obtaining an IBP signal (step 221), and obtaining an SpO2 signal (step 231);
> the step of "respectively processing the at least two physiological signals, to obtain physiological parameters of a same type that are derived from the physiological signals" specifically includes:

respectively processing the ECG signal, the IBP signal, and the SpO2 signal, to obtain an ECG heart rate in the ECG signal, an IBP pulse rate in the IBP, and an SpO2 pulse rate in the SpO2 (step 212, step 222, and step 232);
the step of "respectively analysing the at least two physiological signals, to obtain correlation coefficients of the physiological signals and corresponding historical physiological signals" specifically includes:

respectively analysing the ECG signal, the IBP signal, and the SpO2 signal to calculate a correlation coefficient of an ECG waveform segment and a historical ECG waveform segment, a correlation coefficient of an IBP waveform segment and a historical IBP waveform segment, and a correlation coefficient of an SpO2 waveform segment and a historical SpO2 waveform segment (step 213, step 223, and step 233); and
the step of "fusing, according to the obtained correlation coefficients of the physiological signals and the corresponding historical physiological signals, the physiological parameters of the same type that are derived from the physiological signals, to obtain a fused physiological parameter" specifically includes:

determining a weight of the ECG heart rate according to a correlation coefficient of the ECG signal and a historical ECG signal (step 214);
determining a weight of the IBP pulse rate according to a correlation coefficient of the IBP signal and a historical IBP signal (step 224);
determining a weight of the SpO2 pulse rate according to a correlation coefficient of the SpO2 signal and a historical SpO2 signal (step 234); and
fusing the ECG heart rate and its weight, the IBP pulse rate and its weight, and the SpO2 pulse rate and its weight, so as to obtain a fused heart rate (step 25).

Therefore, in this embodiment, during monitoring of a monitored object, first, the collected ECG signal, IBP signal, and SpO2 signal of the monitored object are respectively analysed, to obtain the ECG heart rate in the ECG signal, the IBP pulse rate in the IBP signal, and the SpO2 pulse rate in the SpO2 signal, and the correlation coefficient of the current ECG signal and the historical ECG signal, the correlation coefficient of the IBP signal and the historical IBP signal, and the correlation coefficient of the SpO2 signal and the historical SpO2 signal are also calculated; and then, the respective weights of the ECG heart rate in the ECG signal, the IBP pulse rate in the IBP, and the SpO2 pulse rate in the SpO2 are determined according to a pre-set correspondence between a correlation coefficient (CR) and a weight (C).

In an embodiment, the pre-set correspondence between a correlation coefficient (CR) and a weight (C) may be as follows:

$$C = \begin{cases} 100 & CR > 90\% \\ 80 & 70\% < CR \leq 90\% \\ 50 & 50\% < CR \leq 70\% \\ 20 & 20\% < CR \leq 50\% \\ 0 & CR \leq 20\% \end{cases}$$

A formula for calculating the final fused heart rate is as follows:

$$HR = \frac{C_{ecg} * HR + C_{ibp} * PR_{ibp} + C_{spo2} * PR_{spo2}}{C_{ecg} + C_{ibp} + C_{spo2}}$$

where HR represents the fused heart rate, $C_{ecg}$ represents the weight of the ECG heart rate, and HR represents the ECG heart rate; $C_{ibp}$ represents the weight of the IBP pulse rate, and $PR_{ibp}$ represents the IBP pulse rate; and $C_{SpO2}$ represents the weight of the SpO2 pulse rate, and $PR_{SpO2}$ represents the SpO2 heart rate.

In another embodiment, the respectively analysing the at least two physiological signals, to obtain correlation coefficients of the physiological signals and corresponding historical physiological signals includes:

obtaining, from the physiological signals, physiological parameters of the same type of the physiological signals within a pre-set time period;
obtaining, from historical signals of the physiological signals, historical trends of the physiological signals within the pre-set time period; and

obtaining, according to a historical trend of the physiological parameters of the same type of the physiological signals and a historical trend of the physiological signals, a fluctuation index (FI) of each physiological signal, and obtaining the correlation coefficient of each physiological signal and the corresponding historical physiological signal according to the fluctuation index.

There may be a predetermined correspondence between the fluctuation index and the correlation coefficient. Therefore, after the fluctuation index is determined, the correlation coefficient can be obtained according to the fluctuation index. For example, in one embodiment, the fluctuation index is a ratio of an absolute value of a difference between the historical trend of the physiological parameters of the same type of the physiological signals and the historical trend of the physiological signals to the historical trend of the physiological signals, and the correlation coefficient is a difference between 1 and the fluctuation index.

Specifically, in a second embodiment, the historical trend of the physiological signals within the pre-set time period includes historical averages of the physiological parameters of the same type that are derived from the historical signals of the physiological signals.

Referring to FIG. 3, in a second specific embodiment, the at least two physiological signals include three physiological signals, which are specifically an ECG signal, an IBP signal, and an SpO2 signal. The three physiological signals are all derived from the periodic beating of the heart. Therefore, an ECG heart rate, an IBP pulse rate, and an SpO2 pulse rate respectively derived from the three physiological signals are strongly correlated with each other, and therefore may be referred to as physiological parameters of the same type.

Therefore, the step of "obtaining at least two physiological signals" specifically includes:

obtaining an ECG signal (step 311), obtaining an IBP signal (step 321), and obtaining an SpO2 signal (step 331);
the step of "respectively processing the at least two physiological signals, to obtain physiological parameters of a same type that are derived from the physiological signals" specifically includes:

respectively processing the ECG signal, the IBP signal, and the SpO2 signal to obtain an ECG heart rate in the ECG signal, an IBP pulse rate in the IBP signal, and an SpO2 pulse rate in the SpO2 signal (step 312, step 322, and step 331);
the respectively analysing the at least two physiological signals, to obtain correlation coefficients of the physiological signals and corresponding historical physiological signals includes:

respectively analysing the ECG signal, the IBP signal, and the SpO2 signal to obtain a fluctuation index of the ECG heart rate and a historical average ECG heart rate and obtain a correlation coefficient according to the fluctuation index (step 313), to obtain a fluctuation index of the IBP pulse rate and a historical average IBP pulse rate and obtain a correlation coefficient according to the fluctuation index (step 323), and to obtain a fluctuation index of the SpO2 pulse rate and a historical average SpO2 pulse rate and obtain a correlation coefficient according to the fluctuation index (step 333); and
the fusing, according to the obtained correlation coefficients of the physiological signals and the corresponding historical physiological signals, the physiological parameters of the same type that are derived from the physiological signals, to obtain a fused physiological parameter includes:

obtaining a weight of the ECG heart rate according to a correlation coefficient of the ECG heart rate and a historical ECG heart rate (step 314);
obtaining a weight of the IBP pulse rate according to a correlation coefficient of the IBP pulse rate and a historical IBP pulse rate (step 324);
obtaining a weight of the SpO2 pulse rate according to a correlation coefficient of the SpO2 pulse rate and a historical SpO2 pulse rate (step 334); and
fusing the ECG heart rate and its weight, the IBP pulse rate and its weight, and the SpO2 pulse rate and its weight, so as to obtain a fused heart rate (step 35).

Specifically, in this embodiment, the fluctuation index of the ECG heart rate and the historical average ECG heart rate is a ratio of an absolute value of a difference between the ECG heart rate and the historical average ECG heart rate to the historical average ECG heart rate. The fluctuation index of the IBP pulse rate and the historical average IBP pulse rate is a ratio of an absolute value of a difference between the IBP pulse rate and the historical average IBP pulse rate to the historical average IBP pulse rate. The fluctuation index of the SpO2 pulse rate and the historical average SpO2 pulse rate is a ratio of an absolute value of a difference between the SpO2 pulse rate and the historical average SpO2 pulse rate to the historical average SpO2 pulse rate.

Therefore, in the second specific embodiment, the ECG signal, the IBP signal, and the SpO2 signal of a monitored object are obtained, and the ECG signal, the IBP signal, and the SpO2 signal are respectively analysed to obtain the ECG heart rate in the ECG signal, the IBP pulse rate in the IBP signal, and the SpO2 pulse rate in the SpO2 signal; a historical trend of the ECG heart rate, a historical trend of the IBP pulse rate, and a historical trend of the SpO2 pulse rate are further respectively obtained from the ECG signal, the IBP signal, and the SpO2 signal; then, a fluctuation index of the ECG heart rate and its historical trend is calculated according to the ECG heart rate and its historical trend, a correlation coefficient is obtained according to the fluctuation index, and the weight of the ECG heart rate is determined according to the correlation coefficient; then, a fluctuation index of the IBP pulse rate and its historical trend is calculated according to the IBP pulse rate and its historical trend, a correlation coefficient is obtained according to the fluctuation index, and the weight of the IBP pulse rate is determined according to the correlation coefficient; then, a fluctuation index of the SpO2 pulse rate and its historical trend is calculated according to the SpO2 pulse rate and its historical trend, a correlation coefficient is obtained according to the fluctuation index, and the weight of the SpO2 pulse rate is determined according to the correlation coefficient; and finally, a fused heart rate is obtained according to the ECG heart rate, the IBP pulse rate, and the SpO2 pulse rate as well as their respective weights.

In a third embodiment, the historical trend of the physiological signals within the pre-set time period includes a historical average of a fused physiological parameter obtained from the historical signals of the physiological signals.

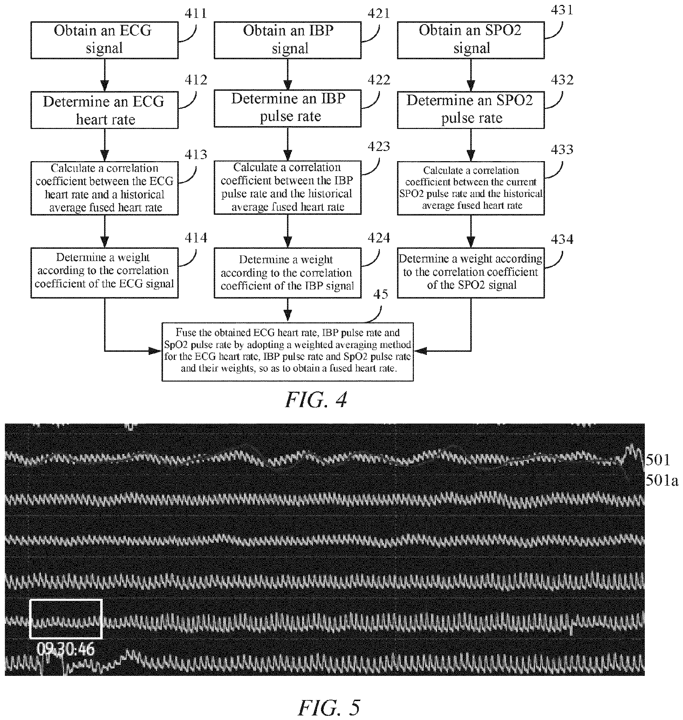

Referring to FIG. 4, the at least two physiological signals include three physiological signals, which are specifically an ECG signal, an IBP signal, and an SpO2 signal. The three physiological signals are all derived from the periodic beating of the heart. Therefore, an ECG heart rate, an IBP pulse rate, and an SpO2 pulse rate respectively derived from the three physiological signals are strongly correlated with each other, so that the ECG heart rate, the IBP pulse rate, and the SpO2 pulse rate may be used as physiological parameters of the same type.

Therefore, the step of "obtaining at least two physiological signals" specifically includes:

> obtaining an ECG signal (step 411), obtaining an IBP signal (step 421), and obtaining an SpO2 signal (step 431);
> the step of "respectively processing the at least two physiological signals, to obtain physiological parameters of a same type that are derived from the physiological signals" specifically includes:
>
>> respectively processing the ECG signal, the IBP signal, and the SpO2 signal to obtain an ECG heart rate in the ECG signal, an IBP pulse rate in the IBP signal, and an SpO2 pulse rate in the SpO2 signal (step 412, step 422, and step 432);
>> the step of "respectively analysing the at least two physiological signals, to obtain correlation coefficients of the physiological signals and corresponding historical physiological signals" specifically includes:
>>
>>> respectively analysing the ECG signal, the IBP signal, and the SpO2 signal to obtain a fluctuation index of the ECG heart rate and a historical average fused heart rate and obtain a correlation coefficient according to the fluctuation index, to obtain a fluctuation index of the IBP pulse rate and the historical average fused heart rate and obtain a correlation coefficient according to the fluctuation index, and to obtain a fluctuation index of the SpO2 pulse rate and the historical average fused heart rate and obtain a correlation coefficient according to the fluctuation index (step 413, step 423, and step 433); and
>>> the step of "fusing, according to the obtained correlation coefficients of the physiological signals and the corresponding historical physiological signals, the physiological parameters of the same type that are derived from the physiological signals, to obtain a fused physiological parameter" specifically includes:
>>>
>>>> obtaining a weight of the ECG heart rate according to a correlation coefficient of the ECG heart rate and the historical average fused heart rate (step 414);
>>>> obtaining a weight of the IBP pulse rate according to a correlation coefficient of the IBP pulse rate and the historical average fused heart rate (step 424);
>>>> obtaining a weight of the SpO2 pulse rate according to a correlation coefficient of the SpO2 pulse rate and the historical average fused heart rate (step 434); and
>>>> fusing the ECG heart rate and its weight, the IBP pulse rate and its weight, and the SpO2 pulse rate and its weight, so as to obtain a fused heart rate (step 45).

Therefore, the physiological signals are respectively analysed first, to obtain the physiological parameters of the same type of the physiological signals; then, the correlation coefficients of the physiological parameters of the same type that correspond to the physiological signals are obtained according to the physiological parameters of the same type of the physiological signals and a historical trend of the physiological parameters of the same type that correspond to the physiological signals; and the fused physiological parameter is obtained according to the correlation coefficients corresponding to the physiological signals.

In a fourth embodiment, the fusion calculation may be used to obtain a fused respiratory rate. It can be understood that the respiratory rate may be directly derived from a RESP signal, or may be calculated from a respiratory wave baseline extracted from an ECG signal, an IBP signal, and an SpO2 signal, and reference may be made to the fusion method in the first specific embodiment for the specific fusion process. The RESP signal can be a signal directly measured by means of thoracic impedance respiration (a respiratory wave is directly obtained). However, the respiratory wave cannot be directly obtained from the ECG signal, the IBP signal, and the SpO2 signal, and the respiratory rate can be calculated only from the respiratory wave baseline extracted from the ECG signal, the IBP signal, and the SpO2 signal (that is, an envelope of an ECG waveform, an IBP waveform, and an SpO2 waveform can be approximately considered as the respiratory wave). Specifically, as shown in FIG. 5, an undulating waveform profile (a part shown by the line 501a) formed after compression of a blood oxygen wave can be approximately considered as a waveform of the respiratory wave.

Optionally, in another embodiment, the respectively analysing the at least two physiological signals, to obtain correlation coefficients of the physiological signals and corresponding historical physiological signals includes:

obtaining, from the physiological signals, physiological parameters of the same type of the physiological signals within a pre-set time period;

obtaining, from historical signals of the physiological signals, physiological parameters of the same type of the historical signals of the physiological signals; and

obtaining a fluctuation index of each physiological parameter according to a statistical relationship between an average and a deviation of each physiological parameter of the same type of each physiological signal and the physiological parameter of the same type of the historical signal of each physiological signal, and obtaining the correlation coefficient of each physiological signal and the corresponding historical physiological signal according to the fluctuation index.

In this way, a fluctuation index of each physiological parameter is obtained according to a statistical relationship between an average and a deviation of each physiological parameter of the same type of each physiological signal and the physiological parameter of the same type of the historical signal of each physiological signal, and the correlation coefficient of each physiological signal and the corresponding historical physiological signal is obtained according to the fluctuation index, such that the method for calculating a correlation coefficient can be adjusted depending on actual situations.

Optionally, in another embodiment, the respectively analysing the at least two physiological signals, to obtain correlation coefficients of the physiological signals and corresponding historical physiological signals includes: determining a number of a noise alarm of each physiological signal within a pre-set time period according to each physiological signal and a historical signal of each physiological signal, and determining the correlation coefficient of each physiological signal and the corresponding historical physiological signal according to the number of the noise alarm of each physiological signal.

In this way, the correlation coefficient of each physiological signal and the corresponding historical physiological signal can be determined according to the number of the noise alarm of each physiological signal, such that the method for calculating a correlation coefficient is diversified.

Optionally, in another embodiment, the respectively analysing the at least two physiological signals, to obtain correlation coefficients of the physiological signals and corresponding historical physiological signals includes: determining, according to each physiological signal and a historical signal of each physiological signal, a number of time during which each parameter of the same type is invalid within a pre-set time period, and determining, according to the number of time during which each parameter of the same type is invalid, the correlation coefficient of each physiological signal and the corresponding historical physiological signal.

In this way, the correlation coefficient of each physiological signal and the corresponding historical physiological signal can be determined according to the number of time during which each parameter of the same type is invalid, such that the method for calculating a correlation coefficient is diversified.

In one embodiment, the parameter processing method further includes the following step: outputting a critical alarm when any of the at least two physiological parameters of the same type of the at least two physiological signals reaches a pre-set critical threshold.

In this way, quick responses can be made to critical states in different situations.

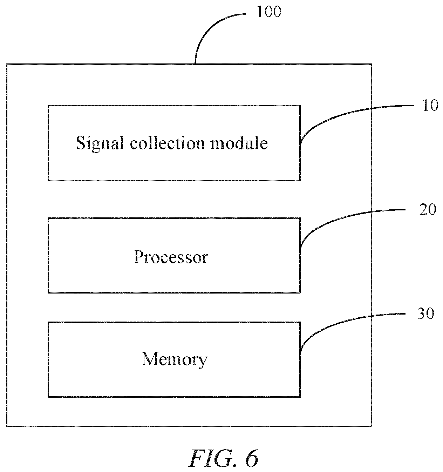

Referring to FIG. 6, FIG. 6 is a schematic modular diagram of a monitoring device according to an embodiment of the disclosure. The monitoring device 100 includes a signal collection module 10, a processor 20, and a memory 30. The signal collection module 10 is configured to collect at least two physiological signals of a monitored object. The memory 30 stores a computer program executable on the processor. The processor 20 executes the computer program to perform the steps of the physiological parameter processing method described above.

An embodiment of the disclosure further provides a computer storage medium for storing a computer program, where the computer program causes a computer to perform some or all of the steps of any one of the physiological parameter processing methods stated in the method embodiments described above.

An embodiment of the disclosure further provides a computer program product, which includes a non-transitory computer-readable storage medium storing a computer program, where the computer program is operable to cause a computer to perform some or all of the steps of any one of the physiological parameter processing methods stated in the method embodiments described above.

It should be noted that, for brevity, the foregoing method embodiments are described as a series of action combinations. However, persons skilled in the art should understand that the disclosure is not limited to the described action order, because according to the disclosure, some steps may be performed in another order or simultaneously. Moreover, persons skilled in the art should also understand that the embodiments described in the specification all are preferred embodiments, and the involved actions and modules are not necessarily required by the disclosure.

In the above embodiments, the embodiments are described with different emphases, and for a part which is not detailed in an embodiment, reference can be made to the related description of the other embodiments.

In several embodiments provided in the disclosure, it should be understood that the disclosed monitoring device may be implemented in other ways. For example, the apparatus embodiments described above are merely exemplary. For example, the division of units is only a logic function division. In actual implementation, there may be other division methods, for example, multiple units or components may be combined or integrated into another system, or some features may be omitted or not implemented. In addition, the mutual coupling or direct coupling or communication connection shown or discussed may be indirect coupling or communication connection through some interfaces, apparatuses, or units, and may be in electrical or other forms.

The units described as separate parts may or may not be physically separated, and the parts displayed as units may or may not be physical units, that is, may be located in one place, or may be distributed over multiple network units. Some or all of the units can be selected according to actual needs to achieve the objectives of solutions of the embodiments.

In several embodiments provided in the disclosure, the processor may be, but is not limited to, a central processing unit (CPU), another general-purpose processor, a digital signal processor (DSP) on a modem, an application specific integrated circuit (ASIC), a field-programmable gate array (FPGA), or another programmable logic device, a discrete gate or transistor logic device, a discrete hardware component, etc.

The functional units in the embodiments of the disclosure may be integrated into one processing unit or may exist as being physically separate, or at least two of the units may be integrated into one unit. The integrated units described above may be implemented in the form of hardware or software program modules.

If the integrated unit is implemented in the form of the software program modules and sold or used as an independent product, it may be stored in a computer-readable memory. Based on such an understanding, the technical solution of the disclosure essentially, or a part contributing to the prior art, or all or part of the technical solution may be embodied in the form of a software product. The computer software product is stored in a memory and includes several instructions to cause a computer device (which may be a personal computer, a server, or a network device, etc.) to execute all or some of the steps of the methods described in the embodiments of the disclosure. The foregoing memory includes: a U disk, a read-only memory (ROM), a random access memory (RAM), a removable hard disk, a magnetic disk or an optical disk and other media that can store program code.

Those of ordinary skill in the art can understand that all or some of the steps of the methods in the above embodiments can be implemented by a program instructing related hardware. The program may be stored in a computer-readable memory, and the memory may include: a flash disk, a read-only memory (ROM for short), a random access memory (RAM for short), a magnetic disk or an optical disk, etc.

The embodiments of the disclosure have been described above in detail, specific examples are used herein to explain the principles and embodiments of the disclosure, and the description of the embodiments is merely intended to facilitate understanding of the method of the disclosure and the core idea thereof. Moreover, for those skilled in the art, changes can be made to the specific embodiments and the scope of application thereof based on the idea of the disclosure. In conclusion, the content of the description should not be construed as limiting the disclosure.

**Claims**

1. A physiological parameter processing method, **characterized by** comprising:

   obtaining at least two physiological signals;
   respectively processing each physiological signal, to obtain a physiological parameter that is respectively derived from each physiological signal, wherein the obtained physiological parameters of the at least two physiological signals are of a same type;
   respectively analysing each physiological signal, to obtain a correlation coefficient between each physiological signal and its corresponding historical physiological signal; and
fusing the obtained physiological parameters of the same type to obtain a fused physiological parameter, according to the obtained correlation coefficients.

2. The physiological parameter processing method of claim 1, **characterized in that**, fusing the obtained physiological parameters of the same type to obtain a fused physiological parameter, according to the obtained correlation coefficients comprises:

    determining a weight of each physiological parameter of the same type, according to each correlation coefficient corresponding to each physiological signal; and
    fusing the obtained physiological parameters of the same type by adopting a weighted averaging method for the at least two physiological parameters of the same type and their at least two weights, so as to obtain the fused physiological parameter.

3. The physiological parameter processing method of claim 2, **characterized in that**, determining a weight of each physiological parameter of the same type, according to each correlation coefficient corresponding to each physiological signal comprises:

    obtaining a pre-set correspondence table of correlation coefficients and weights; and
    obtaining a weight of each physiological parameter of the same type from the correspondence table, according to each correlation coefficients.

4. The physiological parameter processing method of claim 3, **characterized in that**, a weight of a physiological parameter of the same type from a physiological signal with a higher correlation coefficient is greater than a weight of a physiological parameter of the same type from a physiological signal with a lower correlation coefficient.

5. The physiological parameter processing method of any of claims 1 to 4, **characterized in that**, respectively analysing each physiological signal, to obtain a correlation coefficient between each physiological signal and its corresponding historical physiological signal comprises:

    obtaining a waveform segment of each physiological signal that is used to obtain the physiological parameter of the same type; and
    respectively performing a correlation coefficient calculation, according to each waveform segment and a historical physiological signal corresponding to each physiological signal, so as to obtain a correlation coefficient between each physiological signal and its corresponding historical physiological signal.

6. The physiological parameter processing method of claim 5, **characterized in that**, respectively performing a correlation coefficient calculation, according to each waveform segment and a historical physiological signal corresponding to each physiological signal, so as to obtain a correlation coefficient between each physiological signal and its corresponding historical physiological signal comprises:

    obtaining a historical waveform segment of each physiological signal that is used to obtain a historical physiological parameter of a same type from the historical physiological signal of each physiological signal;
    obtaining a fluctuation index of each physiological signal, according to a similarity between the waveform segment of each physiological signal and the historical waveform segment of each physiological signal, and
    obtaining a correlation coefficient between each physiological signal and its corresponding historical physiological signal, according to the fluctuation index of each physiological signal.

7. The physiological parameter processing method of any of claims 1 to 4, **characterized in that**, respectively analysing each physiological signal, to obtain a correlation coefficient between each physiological signal and its corresponding historical physiological signal comprises:

    obtaining, from each physiological signal, a physiological parameter of the same type of each physiological signal within a first pre-set time period;
    obtaining, from a historical physiological signal of each physiological signal, a historical trend of each physiological parameter of the same type of each physiological signal within a second pre-set time period; and
    obtaining a fluctuation index of each physiological parameter of the same type, according to a similarity between each physiological parameter of the same type of each physiological signal and a historical trend of each physiological parameter of the same type of a historical physiological signal of each physiological signal, and obtaining a correlation coefficient between each physiological signal and its corresponding historical physiological signal, according to the fluctuation index of each physiological parameter of the same type.

8. The physiological parameter processing method of claim 7, **characterized in that**, the historical trend of each physiological parameter of the same type comprise a historical average of each physiological parameter of the same type, or a historical average of a fused physiological parameter obtained from the historical physiological signals of the at least two physiological signals.

9. The physiological parameter processing method of any of claims 1 to 4, **characterized in that**, respectively analysing each physiological signal, to obtain a correlation coefficient between each physiological signal and its corresponding historical physiological signal comprises:

obtaining, from each physiological signal, a physiological parameter of the same type of each physiological signal within a pre-set time period;
obtaining, from a historical physiological signal of each physiological signal, a physiological parameter of the same type of the historical physiological signal of each physiological signal; and
obtaining a fluctuation index of each physiological parameter, according to a statistical relationship between an average and a deviation of each physiological parameter of the same type of each physiological signal and each physiological parameter of the same type of the historical physiological signal of each physiological signal, and
obtaining a correlation coefficient between each physiological signal and its corresponding historical physiological signal, according to the fluctuation index of each physiological parameter.

10. The physiological parameter processing method of any of claims 1 to 4, **characterized in that**, respectively analysing each physiological signal, to obtain a correlation coefficient between each physiological signal and its corresponding historical physiological signal comprises:

determining a number of a noise alarm of each physiological signal within a pre-set time period, according to each physiological signal and a historical physiological signal of each physiological signal, and
determining a correlation coefficient between each physiological signal and its corresponding historical physiological signal, according to the number of the noise alarm of each physiological signal.

11. The physiological parameter processing method of any of claims 1 to 4, **characterized in that**, respectively analysing each physiological signal, to obtain a correlation coefficient between each physiological signal and its corresponding historical physiological signal comprises:

determining a number of time during which each parameter of the same type is invalid within a pre-set time period, according to each physiological signal and a historical physiological signal of each physiological signal, and
determining a correlation coefficient between each physiological signal and its corresponding historical physiological signal, according to the number of time during which each parameter of the same type is invalid.

12. The physiological parameter processing method of any of claims 1 to 11, **characterized in that**, the at least two physiological signals comprise at least two of an ECG signal, an IBP signal, and an SpO2 signal; and the at least two physiological parameters of the same type are at least two of an ECG heart rate of the ECG signal, an IBP pulse rate of the IBP signal, and an SpO2 pulse rate of the SpO2 signal.

13. The physiological parameter processing method of claim 12, **characterized in that**, respectively processing each physiological signal, to obtain a physiological parameter of a same type comprises:

respectively processing the at least two of the ECG signal, the IBP signal, and the SpO2 signal to obtain the at least two of the ECG heart rate of the ECG signal, the IBP pulse rate of the IBP signal, and the SpO2 pulse rate of the SpO2 signal;
respectively analysing the at least two physiological signals, to obtain at least two correlation coefficients between the at least two physiological signals and their corresponding at least two historical physiological signals comprises:

respectively analysing the at least two of the ECG signal, the IBP signal, and the SpO2 signal to obtain at least two of a first correlation coefficient between an ECG waveform of the ECG signal and a historical ECG waveform of the ECG signal, a second correlation coefficient between an IBP waveform of the IBP signal and a historical IBP waveform of the IBP signal, and a third correlation coefficient between an SpO2 waveform of the SpO2 signal and a historical SpO2 waveform of the SpO2 signal; and

fusing the at least two physiological parameters of the same type to obtain a fused physiological parameter, according to the obtained at least two correlation coefficients comprises:

obtaining a fused heart rate, according to the at least two of the first correlation coefficient, the second correlation coefficient, and the third correlation coefficient.

14. The physiological parameter processing method of claim 12, **characterized in that**, respectively processing the at least two physiological signals, to obtain at least two physiological parameters of a same type that are respectively derived from the at least two physiological signals comprises:

respectively processing the at least two of the ECG signal, the IBP signal, and the SpO2 signal to obtain at least two of the ECG heart rate of the ECG signal, the IBP pulse rate of the IBP signal, and the SpO2 pulse rate of the SpO2 signal;

respectively analysing the at least two physiological signals, to obtain at least two correlation coefficients between the at least two physiological signals and their corresponding at least two historical physiological signals comprises:

respectively analysing the at least two of the ECG signal, the IBP signal, and the SpO2 signal to obtain at least two of a first correlation coefficient between the ECG heart rate of the ECG signal and a historical average ECG heart rate of the ECG signal, a second correlation coefficient between the IBP pulse rate of the IBP signal and a historical average IBP pulse rate of the IBP signal, and a third correlation coefficient between the SpO2 pulse rate of the SpO2 signal and a historical SpO2 pulse rate of the SpO2 signal; and

fusing the at least two physiological parameters of the same type that are respectively derived from the at least two physiological signals, according to the obtained at least two correlation coefficients between the at least two physiological signals and their corresponding at least two historical physiological signals, to obtain a fused physiological parameter comprises:

fusing at least two of the ECG heart rate of the ECG signal, the IBP pulse rate of the IBP signal, and the SpO2 pulse rate of the SpO2 signal, according to at least two of the first correlation coefficient between the ECG heart rate of the ECG signal and the historical average ECG heart rate of the ECG signal, the second correlation coefficient between the IBP pulse rate of the IBP signal and the historical average IBP pulse rate of the IBP signal, and the third correlation coefficient between the SpO2 pulse rate of the SpO2 signal and the historical average SpO2 pulse rate of the SpO2 signal, so as to obtain a fused heart rate.

15. The physiological parameter processing method of claim 12, **characterized in that**, respectively processing the at least two physiological signals, to obtain at least two physiological parameters of the same type that are respectively derived from the at least two physiological signals comprises:

respectively processing at least two of the ECG signal, the IBP signal, and the SpO2 signal to obtain at least two of the ECG heart rate of the ECG signal, the IBP pulse rate of the IBP signal, and the SpO2 pulse rate of the SpO2 signal;

respectively analysing the at least two physiological signals, to obtain at least two correlation coefficients between the at least two physiological signals and their corresponding at least two historical physiological signals comprises:

respectively analysing the at least two of the ECG signal, the IBP signal, and the SpO2 signal to obtain at least two of a first correlation coefficient between the ECG heart rate of the ECG signal and a historical average fused heart rate, a second correlation coefficient between the IBP pulse rate of the IBP signal and the historical average fused heart rate, and a third correlation coefficient between the SpO2 pulse rate of the SpO2 signal and the historical average fused heart rate; and

fusing the at least two physiological parameters of the same type that are respectively derived from the at least two physiological signals, according to the obtained at least two correlation coefficients between the at least two physiological signals and their corresponding at least two historical physiological signals, to obtain a fused physiological parameter comprises:

fusing the at least two of the ECG heart rate of the ECG signal, the IBP pulse rate of the IBP signal, and the SpO2 pulse rate of the SpO2 signal, according to at least two of the first correlation coefficient between the ECG heart rate of the ECG signal and the historical average fused heart rate, the second correlation coefficient between the IBP pulse rate of the IBP signal and the historical average fused heart rate, and the third correlation coefficient between the SpO2 pulse rate of the SpO2 signal and the historical average fused heart rate, so as to obtain a fused heart rate.

```
                                                                              11

┌──────────────────────────────────────────────────────────┐
│                                                            │
│          Obtain at least two physiological signals         │
│                                                            │
└──────────────────────────────────────────────────────────┘
                             │
                             ▼
                                                                              12
┌──────────────────────────────────────────────────────────┐
│     Respectively process each physiological signal, to obtain │
│         a physiological parameter, wherein the obtained    │
│     physiological signals of the at least two physiological │
│              signals are of a same type                    │
└──────────────────────────────────────────────────────────┘
                             │
                             ▼
                                                                              13
┌──────────────────────────────────────────────────────────┐
│    Respectively analyse each physiological signal, to obtain │
│       a correlation coefficient between each physiological │
│           signal and its corresponding historical         │
│                  physiological signal                     │
└──────────────────────────────────────────────────────────┘
                             │
                             ▼
                                                                              14
┌──────────────────────────────────────────────────────────┐
│        Fuse the obtained physiological parameters of the  │
│     same type to obtain a fused physiological parameter,  │
│       according to the obtained correlation coefficients  │
└──────────────────────────────────────────────────────────┘
```

*FIG. 1*

211 — Obtain an ECG signal

212 — Determine an ECG heart rate

213 — Calculate a correlation coefficient between the current ECG waveform and a historical ECG waveform

214 — Determine a weight according to the correlation coefficient of the ECG signal

221 — Obtain an IBP signal

222 — Determine an IBP pulse rate

223 — Calculate a correlation coefficient between the current IBP waveform and a historical IBP waveform

224 — Determine a weight according to the correlation coefficient of the IBP signal

231 — Obtain an SpO2 signal

232 — Determine an SpO2 pulse rate

233 — Calculate a correlation coefficient between the current SpO2 waveform and a historical SpO2 waveform

234 — Determine a weight according to the correlation coefficient of the SpO2 signal

25 — Fuse the obtained ECG heart rate, IBP pulse rate and SpO2 pulse rate by adopting a weighted averaging method for the ECG heart rate, IBP pulse rate and SpO2 pulse rate and their weights, so as to obtain a fused heart rate.

*FIG. 2*

311 — Obtain an ECG signal

312 — Determine an ECG heart rate

313 — Calculate a correlation coefficient between the ECG heart rate and a historical average ECG heart rate

314 — Determine a weight according to the correlation coefficient of the ECG signal

321 — Obtain an IBP signal

322 — Determine an IBP pulse rate

323 — Calculate a correlation coefficient between the IBP pulse rate and a historical average IBP pulse rate

324 — Determine a weight according to the correlation coefficient of the IBP signal

331 — Obtain an SPO2 signal

332 — Determine an SPO2 pulse rate

333 — Calculate a correlation coefficient between the current SPO2 pulse rate and a historical SPO2 pulse rate

334 — Determine a weight according to the correlation coefficient of the SPO2 signal

35 — Fuse the obtained ECG heart rate, IBP pulse rate and SpO2 pulse rate by adopting a weighted averaging method for the ECG heart rate, IBP pulse rate and SpO2 pulse rate and their weights, so as to obtain a fused heart rate.

*FIG. 3*

```
┌─────────────────────┐ 411    ┌─────────────────────┐ 421    ┌─────────────────────┐ 431
│   Obtain an ECG     │ ⌐/     │   Obtain an IBP     │ ⌐/     │   Obtain an SPO2    │ ⌐/
│      signal         │        │      signal         │        │      signal         │
└─────────────────────┘        └─────────────────────┘        └─────────────────────┘
          │                              │                              │
          ▼                 412          ▼                 422          ▼                 432
┌─────────────────────┐ ⌐/     ┌─────────────────────┐ ⌐/     ┌─────────────────────┐ ⌐/
│  Determine an ECG   │        │   Determine an IBP  │        │  Determine an SPO2  │
│     heart rate      │        │     pulse rate      │        │     pulse rate      │
└─────────────────────┘        └─────────────────────┘        └─────────────────────┘
          │                 413          │                 423          │                 433
          ▼                 ⌐/           ▼                 ⌐/           ▼                 ⌐/
┌─────────────────────┐        ┌─────────────────────┐        ┌─────────────────────┐
│ Calculate a correlation│      │ Calculate a correlation│      │ Calculate a correlation│
│coefficient between the ECG│   │coefficient between the IBP│   │coefficient between the current│
│ heart rate and a historical│  │ pulse rate and the historical│ │SPO2 pulse rate and the historical│
│ average fused heart rate│     │ average fused heart rate│     │ average fused heart rate│
└─────────────────────┘        └─────────────────────┘        └─────────────────────┘
          │                 414          │                 424          │                 434
          ▼                 ⌐/           ▼                 ⌐/           ▼                 ⌐/
┌─────────────────────┐        ┌─────────────────────┐        ┌─────────────────────┐
│  Determine a weight │        │  Determine a weight │        │Determine a weight according│
│according to the correlation│  │according to the correlation│  │to the correlation coefficient│
│coefficient of the ECG signal│ │coefficient of the IBP signal│ │   of the SPO2 signal │
└─────────────────────┘        └─────────────────────┘   45   └─────────────────────┘
          │                              │            ⌐/                │
          │                              ▼                              │
          │      ┌──────────────────────────────────────────────┐      │
          └─────▶│ Fuse the obtained ECG heart rate, IBP pulse rate and│◀─────┘
                 │SpO2 pulse rate by adopting a weighted averaging method│
                 │for the ECG heart rate, IBP pulse rate and SpO2 pulse rate│
                 │ and their weights, so as to obtain a fused heart rate.│
                 └──────────────────────────────────────────────┘
```

*FIG. 4*

*FIG. 5*

100

10

Signal collection module

20

Processor

30

Memory

*FIG. 6*

Europäisches
Patentamt

European
Patent Office

Office européen
des brevets

# EUROPEAN SEARCH REPORT

Application Number

EP 21 19 1412

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2016/192887 A1 (SUN ZE-HUI [CN] ET AL) 7 July 2016 (2016-07-07) * paragraphs [0035] – [0038], [0043], [0098], [0121], [0126], [0127], [0154] * * Equation 1 * | 1–15 | INV. A61B5/1455 A61B5/0215 A61B5/0245 A61B5/00 A61B5/352 |
| Y | WO 2013/036718 A1 (ISIS INNOVATION [GB]; CLIFFORD GARI [GB] ET AL.) 14 March 2013 (2013-03-14) * paragraphs [0026], [0095], [0097], [0105], [0106] * | 1–15 | |
| A | WO 01/25802 A2 (NTC TECHNOLOGY INC [US]) 12 April 2001 (2001-04-12) * the whole document * | 1–15 | |
| A | US 2010/324384 A1 (MOON JIM [US] ET AL) 23 December 2010 (2010-12-23) * the whole document * | 1–15 | |
| A | US 2019/110755 A1 (CAPODILUPO JOHN VINCENZO [US] ET AL) 18 April 2019 (2019-04-18) * the whole document * | 1–15 | TECHNICAL FIELDS SEARCHED (IPC) A61B |

The present search report has been drawn up for all claims

| Place of search | Date of completion of the search | Examiner |
|---|---|---|
| The Hague | 23 December 2021 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 19 1412

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2021

| Patent document cited in search report | | Publication date | Patent family member(s) | | Publication date |
|---|---|---|---|---|---|
| US 2016192887 | A1 | 07-07-2016 | CN | 104434311 A | 25-03-2015 |
| | | | US | 2016192887 A1 | 07-07-2016 |
| | | | WO | 2015035764 A1 | 19-03-2015 |
| WO 2013036718 | A1 | 14-03-2013 | NONE | | |
| WO 0125802 | A2 | 12-04-2001 | AT | 284061 T | 15-12-2004 |
| | | | DE | 60016445 T2 | 08-12-2005 |
| | | | EP | 1224566 A2 | 24-07-2002 |
| | | | US | 6519486 B1 | 11-02-2003 |
| | | | US | 2003009091 A1 | 09-01-2003 |
| | | | US | 2005033129 A1 | 10-02-2005 |
| | | | WO | 0125802 A2 | 12-04-2001 |
| US 2010324384 | A1 | 23-12-2010 | EP | 2442709 A1 | 25-04-2012 |
| | | | SG | 176892 A1 | 30-01-2012 |
| | | | SG | 10201404148W A | 26-09-2014 |
| | | | US | 2010324384 A1 | 23-12-2010 |
| | | | US | 2010324385 A1 | 23-12-2010 |
| | | | US | 2010324386 A1 | 23-12-2010 |
| | | | US | 2010324387 A1 | 23-12-2010 |
| | | | US | 2010324388 A1 | 23-12-2010 |
| | | | US | 2010324389 A1 | 23-12-2010 |
| | | | US | 2014088385 A1 | 27-03-2014 |
| | | | US | 2018042495 A1 | 15-02-2018 |
| | | | US | 2019029534 A1 | 31-01-2019 |
| | | | US | 2019029536 A1 | 31-01-2019 |
| | | | US | 2021251501 A1 | 19-08-2021 |
| | | | WO | 2010148205 A1 | 23-12-2010 |
| US 2019110755 | A1 | 18-04-2019 | EP | 3687392 A2 | 05-08-2020 |
| | | | US | 2019110755 A1 | 18-04-2019 |
| | | | WO | 2019079503 A2 | 25-04-2019 |

EPO FORM P0459

For more details about this annex : see Official Journal of the European Patent Office, No. 12/82